# EUROPEAN PATENT APPLICATION

(11) **EP 4 242 913 A1**
(43) Date of publication of application: **13.09.2023**
(21) Application number: 21888695.0
(22) Date of filing: 08.11.2021
(51) Int. Cl.: G06K 9/62

(54) **DETECTION PLATE HAVING IDENTIFICATION INFORMATION AND ANALYZER FOR READING IDENTIFICATION INFORMATION AND IDENTIFICATION METHOD THEREFOR**

(30) Priority: 09.11.2020 CN 202011238986
(71) Applicant: Leadway (HK) Limited, Hong Kong (CN)
(72) Inventor: CAI, Zhengjun, Hangzhou, Zhejiang 310030 (CN); SHANG, Tao, Hangzhou, Zhejiang 310030 (CN); TANG, Linyong, Hangzhou, Zhejiang 310030 (CN); HU, Haibin, Hangzhou, Zhejiang 310030 (CN); JI, Pinlan, Hangzhou, Zhejiang 310030 (CN); LI, Longqiang, Hangzhou, Zhejiang 310030 (CN); YU, Suyun, Hangzhou, Zhejiang 310030 (CN)
(74) Representative: Schiweck Weinzierl Koch Patentanwälte Partnerschaft mbB
(86) International application number: PCT/CN2021/129361
(87) International publication number: WO 2022/096003

(57) **Abstract**

A detection device having identification information, an analyzer for reading the identification information and an identification method therefor. The detection device includes an upper plate and a bottom plate, which are assembled together to form the housing of the detection device. An identification element (15) for identifying the type of the detection device is disposed on the housing, and the identification element (15) includes at least one identification area, on which at least one recessed portion (152) or one non-recessed portion (151) is disposed. Through cooperation of the identification element (15) disposed on the detection device and a detection switch on the analyzer, i.e., through whether the detection switch of the analyzer is triggered by the identification element (15) or not, the analyzer generates a signal corresponding to whether the corresponding signal is triggered or not to identify the type of the detection device, and the analyzer can automatically identify the type of the detection device in use without the need of selecting the type of the detection device by the user.

## Description

### Field of the Invention

The present invention relates to detection devices for measuring clinical physiological parameter, in particular to a detection device for rapidly measuring biochemical parameter as well as an analyzer for reading identification information and a method for identifying the type of the detection device.

### Background of the Invention

At present, more and more products capable of achieving in situ sampling and obtaining detection results immediately are being investigated and used in clinical applications, and such detections are generally referred to as point-of-care testing (POCT).

For example, a human chorionic gonadotropin (HCG) test strip, a luteinizing hormone (LH) test strip and a follicle-stimulating hormone (FSH) test strip which determine hormone levels. Such test strip generally includes a supporting pad, and a sample addition pad, a label pad, a detection pad and a water absorbing pad position on the supporting pad in sequence from upstream to downstream. Urine added to the sample addition pad flows past the sample addition pad, the label pad, the detection pad and the water absorbing pad sequentially, and the hormones in the sample are fixed in the detection pad by a binding substance on the detection pad. The depth of the color shown on the detection pad in the test strip is linearly correlated with the concentration of the sample to be detected, thus determining the level of the corresponding hormone in the sample. In addition to measurement of hormone levels, there are also test strip for drug type detection, allergen analysis, HBV, HCV, HIV, malaria, dengue fever, influenza, Middle East respiratory syndrome, SARS, 2019-nCoV, and other infectious viruses. With the improvement of people's living standard and the emphasis on their own health management, there are more and more detections for blood glucose, uric acid, blood ketone, cholesterol, lactic acid and other parameters, and test strip suitable for POCT using electrochemical methods, photochemical methods and the like arises correspondingly. For the POCT of urinalysis, there are a plurality of combination ways, for example, test strip for urinalysis detection of 4 parameters, 11 parameters, 14 parameters, etc. The test strip can be directly used for detection, or the test strip can be assembled in one housing.

When the detection is completed using the test strip, the detection result can be obtained by observation with naked eyes or read by an electronic device. For the test strip observed by naked eyes, one can distinguish the detection items by markers provided on the test strip or the housing of the test strip. For using an instrument to read the result, at present, it generally uses the test strip for one item to correspond to one specific instrument. For example, if a woman needs to know her level of HCG, LH, or FSH hormone at home, she needs to not only prepare HCG test strip, LH test strip and FSH test strip, but also equip HCG, LH, and FSH detectors instrument in indoors. This not only results in a waste of detection hardware resources, but also results in that the detection process is cumbersome, as it requires a tester to ensure that the test strip in use and the detector instrument being prepared for measurement correspond to each other. At present, some manufacturers integrate the detection of HCG, LG and FSH on one piece of test strip and use one detector to complete result interpretation. However, while this "one piece of test strip with multiple uses" manner simplifies the process of the tester, it increases the cost of the tester when only one or two of the detection items are required; and in addition, the number of detection items that can be integrated on the test strip is limited, so "one piece of paper with multiple uses" is not suitable for obtaining a large number of detection results. Some other manufacturers provide detection item selection modules on the detector in advance, which requires the tester to manually select the test item corresponding to the test strip. Although this method achieves "one machine with multiple test items", it increases the tediousness of the operation because of the addition of the step of manually selecting the detection item(s) by the tester, and brings problems such as incorrect determination on the detection results due to manual misoperation.

For this reason, there is a great need for an improved solution to simplify the detection operation process and realize more convenient "one machine with multiple test items".

### Summary of the Invention

In order to improve the deficiencies in the prior art, the present invention provides a simple and effective detection device with identification information, and the type of the detection device with identification information can be easily identified by an analyzer for detecting the detection device, and detection is carried out during type identification, so as to achieve the function of one machine with multiple test items. The identification information of the detection device with identification information can also be easily observed by manual work, and can be used for secondary detection to as a fool-proof member.

One of the objective of the present invention is to provide a simple and effective detection device with identification information; the detection device comprises an upper plate and a bottom plate, which are assembled together to form the housing of the detection device; an identification element for identifying the type of the detection device is disposed on the housing, and the identification element comprises a recessed portion, a non-recessed portion, or a combination of the recessed portion and the non-recessed portion.

Preferably, at least two identification elements are comprised, which are disposed at different positions on the detection device, respectively.

Further, the identification elements are disposed at the front end or on the broadside of the housing of the detection device; or the identification elements are disposed at the front end and on the broadside of the housing simultaneously.

Further, one of the two identification elements is set as a recessed portion, and the other one is set as a non-recessed portion; or the two identification elements are both set as recessed portions; or the two identification elements are both set as non-recessed portions.

Further, one identification element is disposed in the upper right corner of the front end of the upper plate and is set as a recessed portion, and the other identification element is disposed in the upper left corner of the front end of the upper plate and is set as a non-recessed portion; or, one identification element is located in the upper right corner of the front end of the upper plate and is set as a non-recessed portion, and the other identification element is located in the upper left corner of the front end of the upper plate and is set as a recessed portion.

Further, the identification element comprises at least one non-recessed portion and one recessed portion, wherein the non-recessed portion contacts a detection switch of the analyzer earlier than the recessed portion during the detection device put into the analyzer.

Further, said identification element is used to trigger the detection switch in the analyzer to identify the type of the detection device.

Further, the type of the detection device is identified in a way that the recessed portion does not trigger the detection switch and/or the non-recessed portion triggers the detection switch.

Further, one identification element on the detection device corresponds to one detection switch on the analyzer.

Further, a test strip is disposed between the upper plate and bottom plate of the detection device.

The test strip can be used to measure hormone levels, drug types, allergens, physiological parameters in urine, HBV, HCV, HIV, malaria, dengue fever, influenza, Middle East Respiratory Syndrome, SARS and 2019-nCoV, blood glucose, uric acid, blood ketone, cholesterol, lactic acid, etc. by using various detection methods such as photochemical methods and electrochemical methods that are suitable for the requirements of POCT.

The present invention further provides an analyzer for reading the identification information of the detection device described in the present invention; the analyzer comprises an upper cover, a bottom cover, a detection device insertion port, and a circuit board assembled between the upper cover and the bottom cover; the detection switch used to distinguish the type of the detection device is mounted to the circuit board; and the position of the detection switch in the analyzer corresponds to the position of the identification element of the detection device inserted into the analyzer. Further, the detection switch in the analyzer is triggered by the non-recessed portion of the identification element of the detection device to enable the analyzer to power on and identify the type of the detection device. Further more, the type of the detection device is identified in a way that the recessed portion does not trigger the detection switch and/or the non-recessed portion triggers the detection switch.

Further, when the detection device is inserted into the analyzer, the non-recessed portion of the identification element of the detection device will touch the button for the detection switch and press the button to trigger the detection switch; and the recessed portion of the identification element of the detection device will not touch the button for the detection switch.

Preferably, the circuit board comprises two detection switches, which are in one-to-one correspondence with the two identification elements of the detection device, respectively. Preferably, the side, facing the detection device, of the button for the detection switch is of a slope surface structure.

Further, the detection switch further has the function of powering on the analyzer.

Further, the circuit board is fixed in the analyzer through a base. A through hole is disposed at a position on the base corresponding to the detection switch; and after the circuit board is assembled to the base, the button for said detection switch passes through the through hole of the base.

On the other hand, the present invention further provides a method for identifying the type of a detection device. The detection switch of the analyzer is triggered by an identification element on the detection device to generate a corresponding signal to identify the type of the detection device, thereby realizing the function of one machine with multiple test items and avoid manual misoperation rate. Moreover, the detection device is easy to operate and convenient to use.

The method for identifying the type of the detection device provided by the present invention comprises a detection device and an analyzer, the detection device comprising a housing formed by assembly of the upper plate and the bottom plate, an identification element disposed on the housing, the analyzer comprising an upper cover, a bottom cover, a detection device insertion port, a circuit board assembled between the upper and bottom covers, and a detection switch mounted to the circuit board to distinguish the type of the detection device, wherein through whether the detection switch of the analyzer is triggered by the identification element or not, the analyzer generates a signal corresponding to whether triggering is carried out or not to identify the type of the detection device.

Further, the identification element comprises a non-recessed portion and a recessed portion, and through the fact that the recessed portion does not trigger the detection switch and/or the non-recessed portion triggers the detection switch, the analyzer generates a signal corresponding to whether triggering is carried out or not to identify the type of the detection device.

Further, when the detection switch is not pressed by the recessed portion to switch on, the analyzer generates a signal 1; when the detection switch is pressed by the non-recessed portion to switch on, the analyzer generates a falling signal 2; and when the detection switch is first pressed by the non-recessed portion to switch on and then is switched off after losing the pressure of the non-recessed portion, the analyzer generates a signal 3 that rises first and then falls.

Further, the analyzer is powered on or waked up from the dormant state through triggering the detection switch of the analyzer by the identification element.

Further, when the detection switch is pressed by the non-recessed portion to switch on, the analyzer is powered on or waked up from the dormant state at the same time.

Further, the detection switch comprises a first detection switch and a second detection switch.

Further, the detection device comprises two identification elements, which correspond to the first detection switch and the second identification switch, respectively; one identification element is located in the upper right corner of the front end of the upper plate and is set as a recessed portion, and the other identification element is located in the upper left corner of the front end of the upper plate and is set as a non-recessed portion; or, one identification element is located in the upper right corner of the front end of the upper plate and is set as a non-recessed portion, and the other identification element is located in the upper left corner of the front end of the upper plate and is set as a recessed portion.

Further, the detection device is inserted into the analyzer, the recessed portion or non-recessed portion of the identification element travels to the detection switch and detects that the first detection switch and/or the second detection switch is pressed, and the singlechip of the analyzer detects the type of the detection device; when both the first detection switch and the second detection switch are pressed, two falling signals 2 are emitted, indicating that the detection device is of a first type; or, when the first detection switch is pressed and the second detection switch is not pressed, a first falling signal 2 is emitted, indicating that the detection device is of a second type; or, when the first detection switch is not pressed and the second detection switch is pressed, a second falling signal 2 is emitted, indicating that the detection device is of a third type; or, when neither the first detection switch nor the second detection switch is pressed, the signal 1 is emitted, indicating that the detection device is of a fourth type.

Further, if the singlechip of the analyzer is in a dormant state before the detection device is inserted into the analyzer. The signal indicating the first or second detection switch is triggered will wake up the singlechip, and then the singlechip will analyze the trigger signal from the detection switch, thus distinguishing the detection type of the detection device; and if the singlechip is already in a wake-up state before the detection device is inserted, the singlechip will directly analyze the trigger signal from the detection switch, thus distinguishing the detection type of the detection device. Further, another identification element comprises a non-recessed portion and a recessed portion. The non-recessed portion contacts the detection switch of the analyzer earlier than the recessed portion; the analyzer comprises a detection switch corresponding to the identification element; and when the detection device advances in the analyzer, the detection switch is pressed first and then switched off, a signal 3 that first falls and then rises is emitted, indicating that the detection device is of another type.

The present invention has the beneficial effect that through cooperation of the identification element disposed on the detection device and a detection switch on the analyzer, the analyzer can automatically identify the type of the detection device in use without the need of selecting the type of the detection device by the user, so as to realize the function of one machine with multiple test items. Reduction of operation steps of the user in the detection process can reduce the manual misoperation rate, and it is easy to operate and convenient to use. The present invention can also use a switch system to achieve identification of the types of different detection devices, as well as to achieve the function of waking up the analyzer. The cost of hardware resources is significantly reduced, which brings better use experiences to users. The identification element disposed on the detection device of the present invention not only can cooperate with the analyzer to realize the function of one machine with multiple uses, but also can identify the corresponding type of the detection device by manually identifying the identification element on the detection device, so as to as fool-proof secondarily and ensure accurate acquisition of the detection results.

### Brief Description of the Drawings

Fig. 1 is a schematic diagram of a detection device with covers of the present invention.
Fig. 2 is a schematic diagram of a detection device with no cover of the present invention.
Fig. 3 is an exploded schematic diagram of the detection device in Fig. 2.
Fig. 4 is a schematic diagram that the identification element of the detection device does not contain a recessed portion.
Fig. 5 is a schematic diagram that the identification element of the detection device contains a right recessed portion.
Fig. 6 is a schematic diagram that the identification element of the detection device contains a left recessed portion.
Fig. 7 is a front schematic diagram of the analyzer in mated use with the detection device.
Fig. 8 is an exploded schematic diagram of the analyzer as shown in Fig. 7.
Fig. 9 is a schematic diagram that the circuit board of the analyzer and the detection switch on the circuit board cooperate with a base.
Fig. 10 is a schematic diagram that the detection device of the present invention is located in the slot of the base.
Fig. 11 is a schematic diagram of the correspondence of the detection switch on the circuit board and the identification element on the detection device.
Fig. 12 is a schematic diagram that the button for the first detection switch is not pressed by the identification element and the button for the second detection switch is pressed by the identification element after the detection device is inserted into the analyzer.
Fig. 13 is an enlarged view of A in Fig. 12.
Fig. 14 is a sectional view that the button for the first detection switch is not pressed by the identification element in Fig. 12.
Fig. 15 is an enlarged view of B in Fig. 14.
Fig. 16 is a cross-sectional schematic diagram of cooperation of the detection device and analyzer in Fig. 12.
Fig. 17 is an enlarged view of C in Fig. 16.
Fig. 18 is a front schematic diagram that the identification element is disposed on the broadside of the detection device and does not contain a recessed portion, the button for the detection switch is located on the broadside of the detection switch and the identification element presses the button for the detection switch.
Fig. 19 is a schematic diagram of Fig. 18 from another view.
Fig. 20 is a front schematic diagram that the identification element is disposed on the broadside of the detection device and contains a recessed portion on one broadside, the button for the detection switch is located on the broadside of the detection switch and the identification element cooperates with the button for the detection switch.
Fig. 21 is a schematic diagram of Fig. 19 from another view.
Fig. 22 is a schematic diagram of a switch detection circuit.
Fig. 23 is a flow chart that the analyzer determines the type of the detection device.
Fig. 24 is a schematic diagram of another design of the identification element of the detection device of the present invention.
Fig. 25 is a schematic diagram of another design of the identification element of the detection device of the present invention.
Fig. 26 is signals generated by cooperation of the identification element of the detection device and the detection switch of the present invention. Signal 1 is a signal state that the button for the detection switch is not pressed; signal 2 is a signal state that the button for the detection switch is pressed; signal 3 is a signal state that the button for the detection switch is pressed first and then reset (bounce up).
Fig. 27 is a schematic diagram that the detection device includes only one identification element which is a recessed portion.
Fig. 28 is a schematic diagram that the detection device includes only one identification element which is a non-recessed portion.

### Detailed Description of the Embodiments

The present invention will be described below in detail in conjunction with specific drawings. These specific embodiments are only limited enumeration without departing from the spirit of the present invention, and do not exclude other specific embodiments derived from the combination of the prior art and the present invention by a person of ordinary skill in the art.

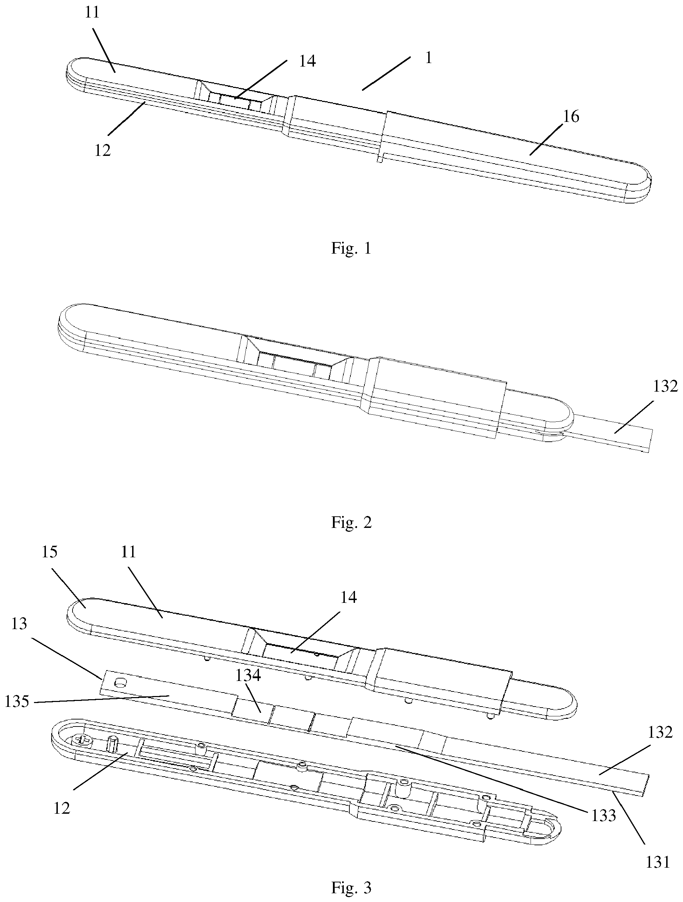

As shown in Figs. 1 to 6, the detection device 1 includes an upper plate 11, a bottom plate 12, test strip 13 assembled between the upper and bottom plates, a test result reading window 14, and an identification element 15 indicating the type of the detection device, the identification element 15 being used to trigger a detection switch in an analyzer 20 to identify the type of the detection device. Specifically, the identification element 15 identifies the type of the detection device through the presence or absence of a recessed portion and the position of the recessed portion, that is, it identifies the type of the detection device in a way that the recessed portion does not trigger the detection switch and/or the non-recessed portion triggers the detection switch. The test strip 13 includes a supporting pad 131, a sample addition pad 132, a label pad 133, a detection pad 134 and a water absorbing pad 135 superposed together and disposed on the supporting pad in sequence from upstream to downstream. After a liquid sample is added to the sample addition pad, the sample flows past the sample addition pad, the label pad, the detection pad and the water absorbing pad sequentially. The test strip is mounted between the upper plate and the bottom plate, and a part of the sample addition pad is located outside the upper and bottom plates to receive the liquid sample. The detection device may further include a cover 16 to cover and protect the sample addition pad exposed to the outside.

The identification element includes a recessed portion, a non-recessed portion, or a combination of the recessed portion and the non-recessed portion. The type of the detection device is identified through the number and combination of the recessed portion and non-recessed portion in the identification element, and thus to achieve the purpose of identifying the detection type of the detection device. The identification element is provided with at least one recessed portion and/or at least one non-recessed portion.

The recessed portion and non-recessed portion in the identification element are arranged in many ways, including but not limited to: first, the identification element includes only the non-recessed portion(s), that is, the identification element is one or more non-recessed portions; second, the identification element includes the recessed portion(s), that is, the identification element is one or more non-recessed portions; third, the identification element includes both the recessed portion(s) and the non-recessed portion(s), that is, one or more recessed portions and non-recessed portions are provided, etc.

The number of the identification element(s) may be one or at least two, for example, two, three, four, etc. The identification element is located on the detection device, particularly on the housing of the detection device, i.e., the identification element is located on the housing formed by closure of the upper plate and the bottom plate. The identification elements are disposed at the front end or on broadside of the housing of the detection device, or are disposed at the front end and on the broadside of the housing simultaneously. The front end described here includes the upper and bottom surfaces of the housing, and also includes the part protruding from the front end of the housing.

There are many ways of combination of the identification elements, including but not limited to: 1, two identification elements are comprised, which are disposed at different positions on the detection device, respectively; for example, the two identification elements are disposed at the front end or two side walls of the upper plate, wherein one of the identification elements is set as a recessed portion, and the other one is set as a non-recessed portion; or the two identification elements are both set as recessed portions; or the two identification elements are both set as non-recessed portions. 2. Two identification elements are comprised; one identification element is located in the upper right corner of the front end of the upper plate and is set as a recessed portion, and the other identification element is located in the upper left corner of the front end of the upper plate and is set as a non-recessed portion; or, one identification element is located in the upper right corner of the front end of the upper plate and is set as a non-recessed portion, and the other identification element is located in the upper left corner of the front end of the upper plate and is set as a recessed portion. 3. One or more identification elements are comprised, wherein one identification element includes at least one non-recessed portion and one recessed portion, wherein said non-recessed portion is closer to the front end of the detection device than the recessed portion, and the non-recessed portion contacts a detection switch of the analyzer earlier than the recessed portion. 4. Four identification elements are comprised, which are disposed at the front end and two sides of the detection device, respectively.

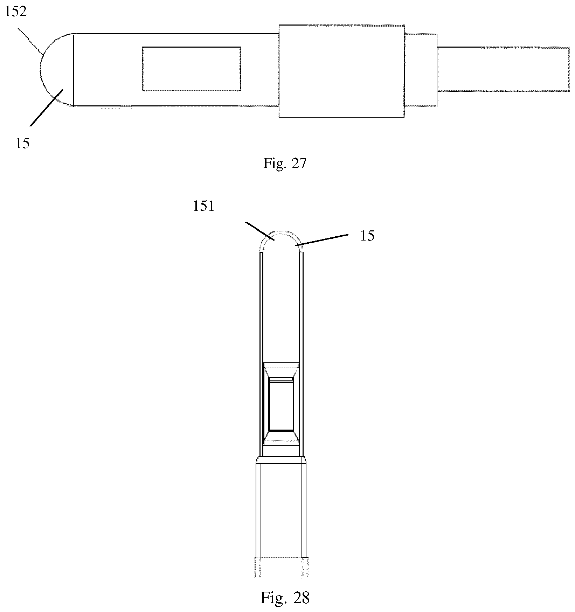

In one design solution, as shown in Figs. 27 and 28, the detection device includes an identification element 15 at the front end of the upper plate 11, there are two setting modes for the identification element: the first mode is that the front end of the upper plate is provided with no recessed portion, i.e., the identification element only includes the non-recessed portion 151, as shown in Fig. 28; the second mode is that the whole front end of the upper plate is set as a recessed portion 152, as shown in Fig. 27.

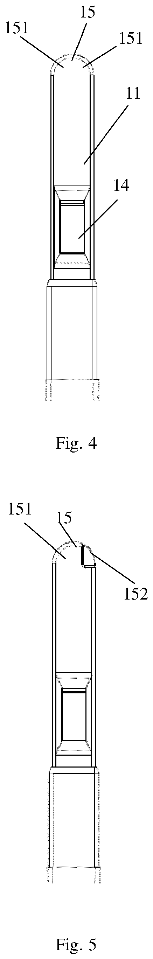

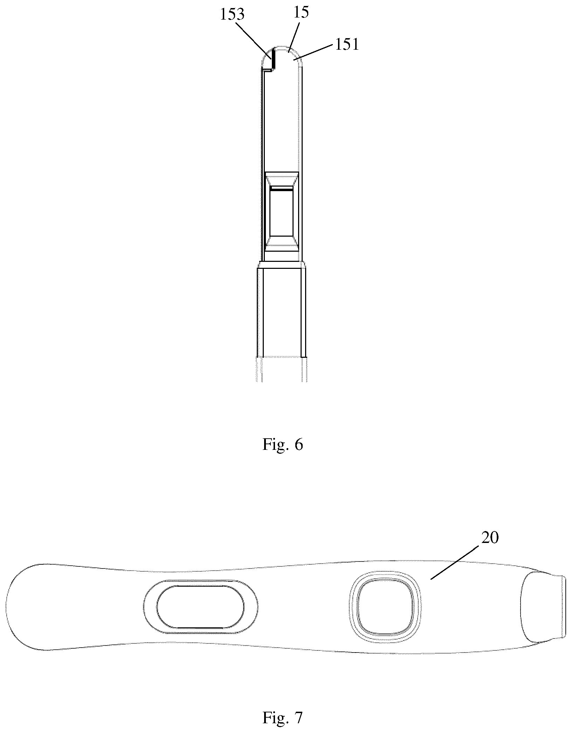

In another design solution, as shown in Figs. 4 to 6, the detection device includes two identification elements 15 located at the front end of the upper board 11. There are three setting modes for the two identification elements: the first mode is that the front end of the upper plate is provided with no recessed portion, that is, both the two identification elements are non-recessed portions, as shown in Fig. 4; the second mode is that there is a recessed portion (the first identification element) in the upper right corner of the upper plate, and there is no recessed portion (the second identification element) in the upper left corner, as shown in Fig. 5; the third mode is that there is no recessed portion (the first identification element) in the upper right corner of the upper plate, and there is a recessed portion (the second identification element) in the upper left corner, as shown in Fig. 6, wherein the second mode and the third mode are the setting way that in the case where the identification elements are set as one recessed portion and one non-recessed portion, the position relationship between the recessed portion and the non-recessed portion is changed.

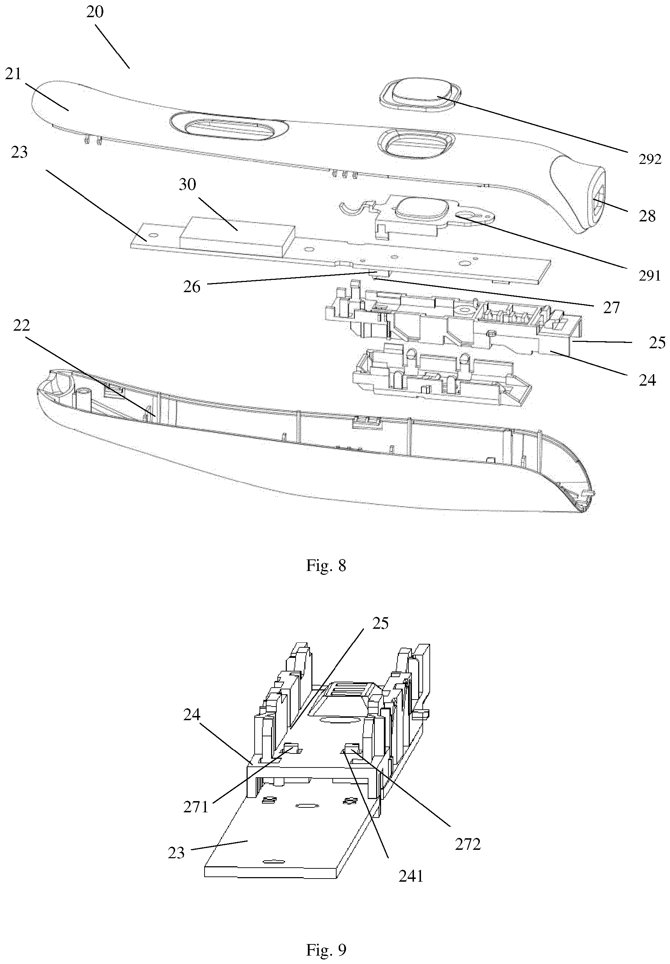

The analyzer 20 used in cooperation with the detection device, as shown in Figs. 7 to 10, includes an upper cover 21, a bottom cover 22, and a circuit board 23 assembled between the upper and bottom covers. The circuit board 23 is fixed in the analyzer through a base 24, the upper cover and the bottom cover are assembled together to form the housing of the analyzer, and a detection device insertion port 28 is formed at the front end. The other side of the base 24 opposed to the circuit board mounting side is provided with a slot 25 for insertion of the detection device. The detection switch 26 used to identify the type of the detection device is mounted on the circuit board, and the base 24 is provided with a through hole 241 at a position corresponding to the detection switch, and after the circuit board is assembled to the base, the button 27 for the detection switch 26 mounted on the circuit board extends through the through hole 241 into the slot 25. After the detection device is inserted into the slot of the analyzer, the identification element 15 of the detection device is located above the button 27 for the detection switch 26. If the part of the identification element 15 corresponding to the detection switch 26 is set as a non-recessed portion, the non-recessed portion of the housing of the detection device will touch the button 27 for the detection switch and press the button to trigger the detection switch 26 to switch on; and if the part of the identification element corresponding to the detection switch is set as a recessed portion, the recessed portion of the housing of the detection device will not touch the button for the detection switch, and the button will not be pressed to trigger the switch to switch on as it is being pressed by the detection device to descend. The shape of the button 27 can be set as a slope, a hemisphere, a rectangle or a square, or the like. For example, the side of the button facing the detection device is of a slope surface structure, which is more beneficial for the detection device to push and press the button after it is pushed into the slot and contacts the button.

The analyzer 20 can be provided with a power-on switch individually. In one design solution, said detection switch 26 for the type of the detection device may also have the function of powering on the analyzer. The detection switch 26 in the analyzer is triggered by the non-recessed portion of the identification element 15 of the detection device to enable the analyzer to power on and identify the type of the detection device. That is, after the detection switch 26 is triggered, it causes the analyzer 20 to power on and start up, and meanwhile the analyzer 20 after starting up can determine the type of the detection device based on the triggering situation of the detection switch.

The analyzer 20 may further include a detection device ejection assembly. The detection device ejection assembly includes an ejection connection block 291 and an ejection button 292 mounted in the housing of the analyzer.

In some design solutions, one identification element on the detection device corresponds to one detection switch on the analyzer. For example, two identification elements are disposed on the detection device, and correspondingly, two detection switches are disposed on the analyzer, and the identification elements are in one-to-one correspondence with the switches in position.

Further specific illustration will be made below with an example of using the different modes of identification elements 15 disposed on the detection device to distinguish an hCG detection device, an FSH detection device and an LH detection device.

The test strip for analyzing the hCG content in urine is stored in the detection device as shown in Fig. 4. No recessed portion is disposed at a position on the identification element 15 of this detection device corresponding to the button 27 for the detection switch 26 of the analyzer, i.e., the identification element at the front end of the upper plate is wholly a non-recessed portion 151. In the example of Fig. 4, said non-recessed portion is the part of the upper surface of the upper plate 11 that extends horizontally to the front end of the upper plate.

Test strip for analyzing the LH content in urine is stored in the detection device as shown in Fig. 5. A right recessed portion 152 (first identification element) is disposed at a position on the identification element 15 of this detection device corresponding to the button 271 for the first detection switch 261 of the analyzer, i.e., a recessed structure is disposed in the upper right corner of the front end of the upper plate; no recessed portion is disposed at a position on the identification element 15 of this detection device corresponding to the button 272 for the second detection switch 262 of the analyzer, i.e., a non-recessed portion 151 (second identification element) is disposed at the upper left corner of the front end of the upper plate.

Test strip for analyzing the FSH content in urine is stored in the detection device as shown in Fig. 6. A left recessed portion 153 is disposed at a position on the identification element 15 of the detection device corresponding to the button for the second detection switch of the analyzer, i.e., a left recessed structure is disposed in the upper left corner of the front end of the upper plate, and no recessed portion is disposed at a position on the identification element 15 of the detection device corresponding to the button for the first detection switch of the analyzer, i.e. a non-recessed portion 151 is disposed in the upper right corner of the front end of the upper plate.

The depth of the recessed portion is set to ensure at least the bottom of this recessed portion does not touch the button for the detection switch.

The type of the detection device can be determined by whether the detection switch of the analyzer is pressed by the identification element of the detection device. The analyzer and the detection switch for the type of the detection device will be described in detail below. The tester can also determine the type of the detection device currently in use by directly observing the identification element of the detection device, instead of using the analyzer. As for the detection device of which the detection type is determined by direct observation of the tester, the position of the recessed portion or non-recessed portion of the identification element may not correspond to the detection switch of the analyzer.

For example, when it is detected by an instrument or observed by human eyes that the identification element of the detection device is wholly non-recessed portions, it determines that the detection device is an HCG detection device; or when it is detected or observed that the identification element of the detection device is only provided with one right recessed portion, it determines that the detection device is anLH detection device; or when it is detected or observed that the identification element of the detection device is only provided with one left recessed portion, it determines that the detection device is an FSH detection device; or when it is detected or observed that the identification element of the detection device is provided with both the right and left recessed portions, it determines that the detection device is an E2 (estradiol) detection device.

The person skilled in the art can provide different number of identification elements on the detection device, different number of recessed portions on the identification elements, and positions of the identification elements and recessed portions on the detection device according to the number of relevant detection items; and accordingly, different number of detection switches are disposed at corresponding positions on the analyzer to correspond to the identification elements. It is also possible to complete the correspondence between the identification elements and the detection types by the number of the recessed portions, the positions of the recessed portions, or the arrangement and combination of the number of the recessed portions and the positions of the recessed portions. The recessed portion may be designed as other structure with similar functions, such as a notch. The LH detection device in Fig. 5 is taken as an example below to further illustrate the identification element of the detection device cooperating with the detection switch of the analyzer to determine the type of the detection device.

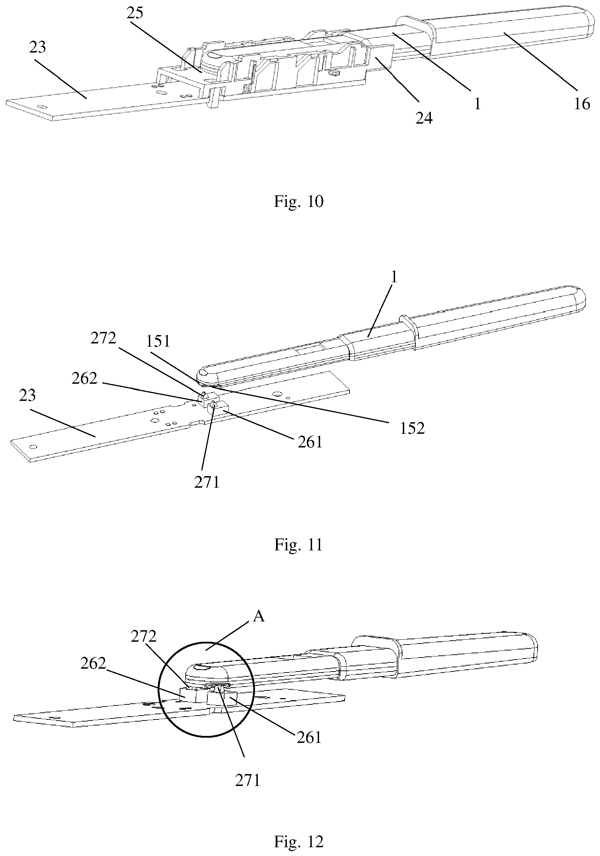

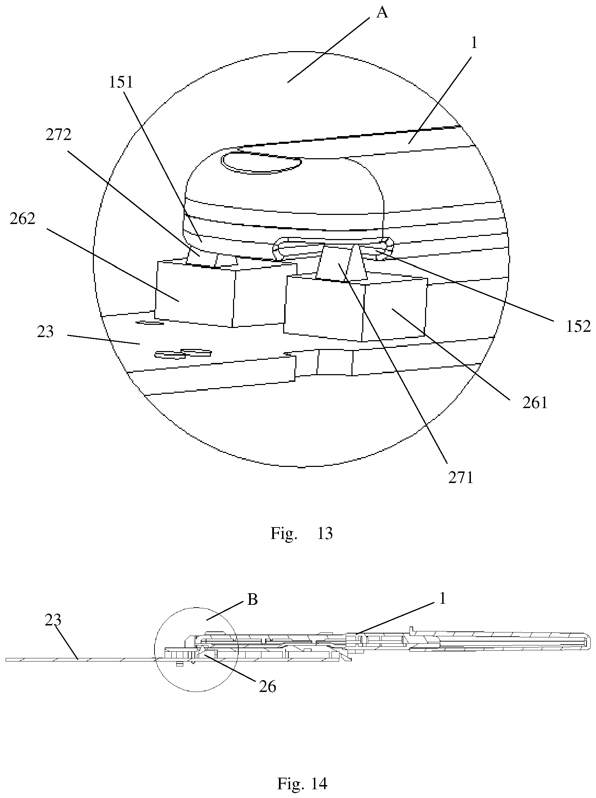

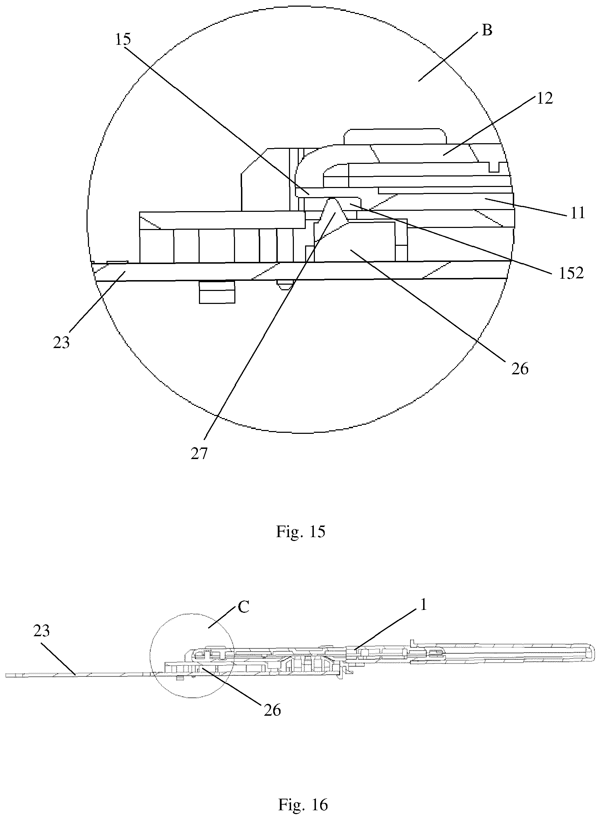

Two detection switches are provided side by side on the circuit board of the analyzer shown in Figs. 11 to 17, which are a first detection switch 261 and a second detection switch 262, respectively. In order to more clearly express the cooperation between the identification element of the detection device and the detection switch of the analyzer, mounting pieces for mounting the circuit board in the housing of the analyzer are omitted in the example drawings (Figs. 11 and 12), for example, the base is omitted in these drawings. When the detection device with a right notch is inserted into the analyzer, the part of the identification element corresponding to the second button 271 for the second detection switch 261 of the analyzer is a right recessed portion 152 (first identification element), and the bottom wall of the recessed portion will not contact the first button 271, and the first button 271 will not be pressed to descend in the process of inserting the detection device to the analyzer, so that the first detection switch 261 cannot be triggered by the first button to switch on, and thus the signal given to the singlechip by the first detection switch 261 is always a high level signal (signal 1). The part of the identification element corresponding to the second button 272 for the second detection switch 262 of the analyzer is a non-recessed portion 151 (second identification element), the plate wall of the non-recessed portion will contact the second button 272, and the second button 272 will be pressed by the non-recessed portion to descend in the process of inserting the detection device into the analyzer, so the second detection switch 262 will be triggered by the second button 272 to convert high level at a pin of the singlechip connected with the second detection switch 262 to low level, thus generating a falling edge signal (signal 2). The analyzer can determine that the inserted detection device is an LH detection device after obtaining no change of the first detection switch 261 and the falling edge signal of the second detection switch 262.

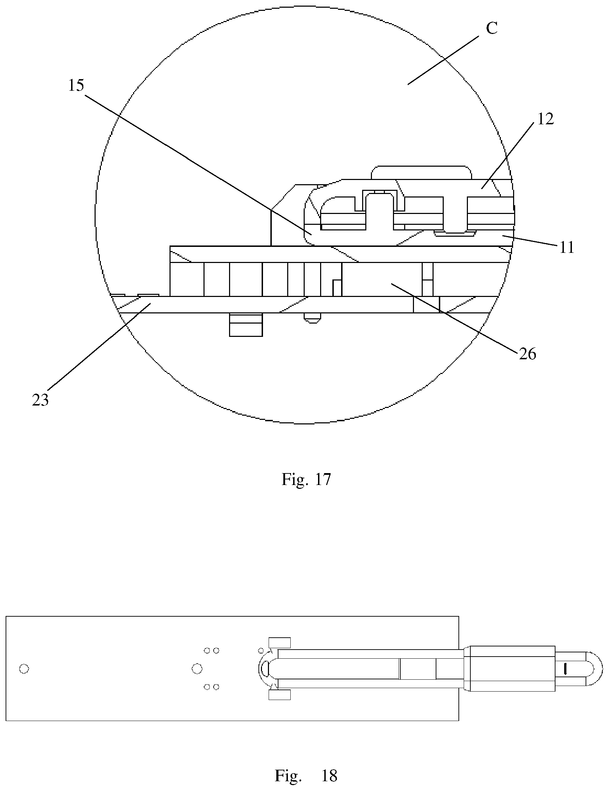

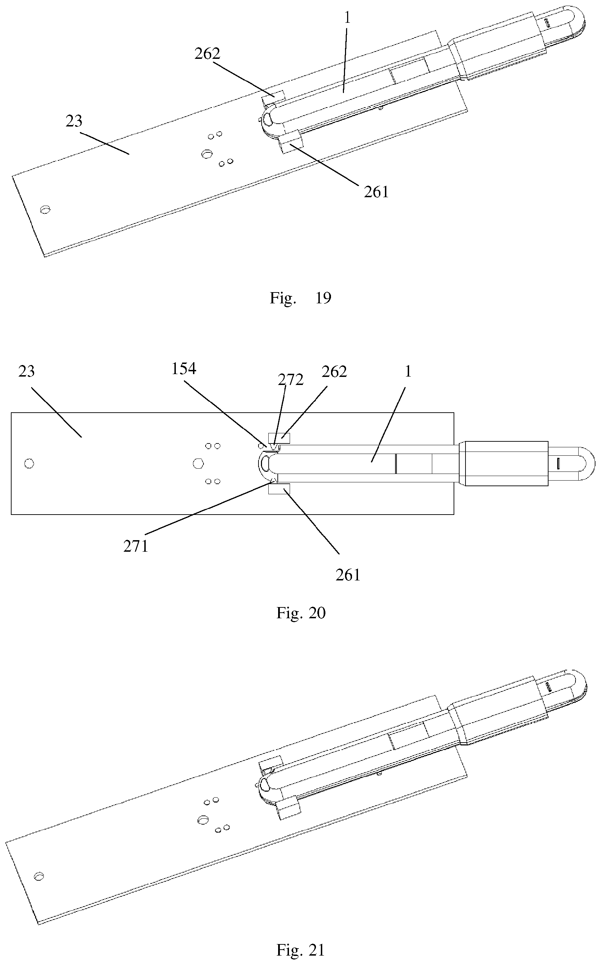

The identification element 15 for the type of the detection device can also be disposed on the broadside of the detection device to determine the type of the detection device through the presence or absence of the recessed portion on the side wall and the position of the recessed portion, as shown in Figs. 18 to 21. The setting modes of the recessed portion include but not limited to the follows ways: neither of the two side walls of the detection device is provided with a recessed portion; or one side wall of the detection device is provided with a recessed portion and the other side wall is provided with no recessed portion; or the two side walls are each provided with a recessed portion, etc. The identification element of the detection device as shown in Figs. 18 and 19 is provided with no recessed portion, and the button for the detection switch is correspondingly mounted to the inner side face of the detection switch. In the process of inserting the detection device 1 into the analyzer, the first detection switch 261 and the second detection switch 262 are triggered by the identification elements of the detection device press the buttons for the switches. As shown in Fig. 20 and Fig. 21, the identification element of the detection device is provided with a recessed portion 154 on one side and is not provided with a recessed portion on the other side. In the process of inserting the detection device 1 into the analyzer, the first detection switch 261 will be triggered by the fact that the button for the switch is pressed by the identification element of the detection device, while the second detection switch 262 will not be triggered since it is not pressed by the identification element of the detection device.

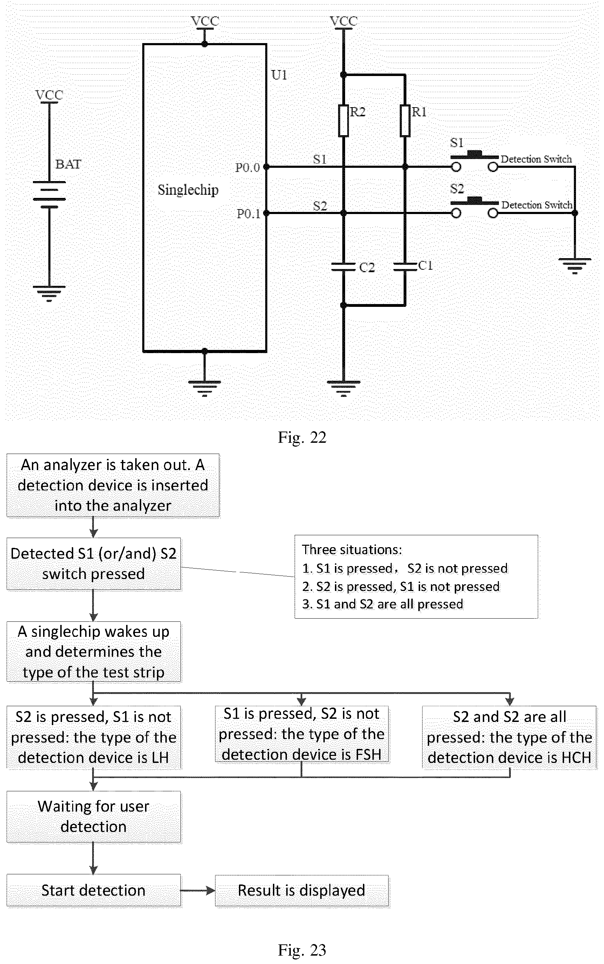

Fig. 22 and Fig. 23 are the working principle of an analyzer of the present invention to identify the type of a detection device. The circuit consists of two branch circuits: a first circuit including a resistor R1, a capacitor C1, a detection switch S1, a VCC power supply terminal, a signal ground and the P0.0 terminal of a singlechip; a second circuit including a resistor R2, a capacitor C2, a detection switch S2, a VCC power supply terminal, a signal ground and the P0.1 terminal of the singlechip. When the detection switch S1 or S2 is not pressed by the button, the voltage across P0.0 and P0.1 of the singlechip is kept in line with the VCC voltage, and the detection switch provides a constant high level signal (signal 1) to the singlechip. When the detection switch S1 or S2 is pressed by the button, P0.0 and P0.1 of the singlechip to which it is connected will be connected to the signal ground, while the voltage across P0.0 and P0.1 will be pulled down by the signal ground and changed from high level to low level, thus generating a falling edge signal (signal 2) to the singlechip.

The power source of the analyzer can be selected from button cells, alkaline cells, rechargeable lithium cells, solar cells, switching power sources, etc.

An analysis method for analyzing an analyte in a sample using the detection device and analyzer described in the present invention, includes the following steps: inserting the detection device into the analyzer; triggering at least one detection switch, the triggered detection switch generates an external trigger signal to give to the singlechip. After receiving this trigger signal, the singlechip generates an external interruption to wake up the singlechip. The singlechip waked up from dormancy gives priority to process the interrupt program triggered by the detection switch to determine the type of test strip in the interrupt program. If the button is not pressed as the detection device ejects the analyzer after the singlechip is waked up or as the detection device is incorrect in mounting position or loosen, the detection switch generates a rising edge signal given to the singlechip, so that the detection device position monitoring program of the singlechip is triggered to inform the singlechip of the abnormal position of the detection device. The singlechip monitors the pressed state of the switch in the detection device position monitoring program to determine whether a test strip is mounted in place and whether it is replaced with a test strip of another type. The sample is added to the test strip; the analyzer selects a corresponding algorithm to read the test result of the test strip according to the type of the detection device; and the analyzer gives the detection result.

The sample analysis method is specifically illustrated by taking the cooperation of the detection device and the analyzer described in Figs. 4 to 6 as an example in combination with Figs. 22 and 23. Step 1: the analyzer and the detection device are taken out, and the detection device is inserted into the slot through the insertion port on the analyzer.

Step 2: with the insertion of the detection device, the non-recessed portion of the identification element of the detection device will press the button for the detection switch S1 and/or S2, the detection switch for which the button is pressed is triggered, and the trigger information of triggering the switches S1 and S2 is transmitted to the singlechip.

When the detection device shown in Fig. 4 is inserted, the buttons for the first detection switch S1 and the second detection switch S2 on the circuit board are pressed together by the detection device, and the singlechip receives a combination of signals that S1 and S2 are both triggered. After signal analysis, the singlechip determines that the detection device currently inserted into the analyzer is for detecting HCG.

When the detection device shown in Fig. 5 is inserted, the button 271 for the first detection switch S1 (261) on the circuit board is not pressed by the detection device, while the button 272 for the second detection switch S2 (262) is pressed by the detection device, and the singlechip receives a combination of signals that S1 is not triggered and S2 is triggered. After signal analysis, the singlechip determines that the detection device currently inserted into the analyzer is for detecting LH.

When the detection device shown in Fig. 6 is inserted, the button for the first detection switch S1 on the circuit board is pressed by the detection device, while the button for the second detection switch S2 is not pressed by the detection device, and the singlechip receives a combination of signals that S1 is triggered and S2 is not triggered. After signal analysis, the singlechip determines that the detection device currently inserted into the analyzer is for detecting FSH.

Step 3: the analyzer starts a test program corresponding to the type of the detection device.

Step 4: the test ends and the test result is displayed.

If the singlechip is in a dormant state before insertion of the detection device, the signal indicating the detection switch is triggered will wake up the singlechip first, and then the singlechip will analyze the trigger signal for the detection switch, thus distinguishing the detection type of the detection device; and

If the singlechip is already in a wake-up state before the detection device is inserted, the singlechip will directly analyze the trigger signal for the detection switch, thus distinguishing the detection type of the detection device.

The analyzer displays the detection type of the detection device on the display screen 30 of the analyzer is an optional and non-essential step.

Detection devices and analyzers vary in design. For some analysis systems, a sample has already been added to the detection device before the detection device is inserted into the analyzer; and for other analysis systems, a sample is added to the detection device after the detection device is inserted into the analyzer and the device type is confirmed.

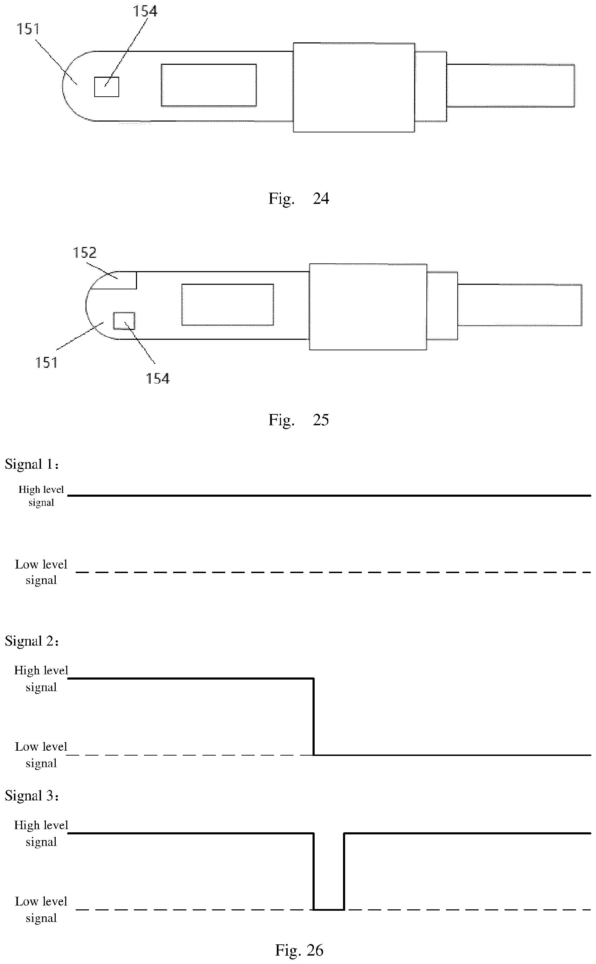

When the detection device described in Figs. 4 to 6 is inserted into the analyzer, two signals, i.e., signal 1 and signal 2 as shown in Fig. 26, are generated. When the recessed portion of the identification element of the detection device cooperates with the detection switch of the analyzer, the analyzer generates signal 1 and transmits it to the singlechip, wherein signal 1 does not generate a rising or falling signal, i.e., signal 1 is a constant high level signal. When the non-recessed portion of the identification element of the detection device cooperates with the detection switch of the analyzer, the analyzer generates signal 2 and transmits it to the singlechip, and when the button for the switch is pressed by the non-recessed region of the identification element, a falling signal 2 is generated.

When the detection switch also has the function of powering on the analyzer, the identification element on the detection device needs to include the non-recessed portion to generate signal 2, and the singlechip wakes up to the power-on function through the change of signal falling of signal 2. If the identification elements on the detection device are both recessed portions, the switch of the analyzer cannot be pressed, so the analyzer cannot determine whether the test strip is already inserted and wake up the power-on function by solely relying on the identification elements, the analyzer needs to add an additional power-on switch.

In order to better realize the detection switch having the function of the power-on switch, in the optimized design of the detection device in Fig. 24, the non-recessed portion 151 and the recessed portion 154 are disposed on one identification element in sequence, and the non-recessed portion contacts the detection switch earlier than the recessed portion 154. When the detection device described in Fig. 24 is inserted into the analyzer, the foremost end of the identification element 15 of the detection device will first press the button for the detection switch 26. Subsequently, insertion of the detection device continues until it is inserted in place, the detection switch is precisely located in the recessed portion 154 of the detection device shown in Fig. 24, and the detection switch 26 which was previously pressed by the non-recessed portion 151 will be ejected again. Throughout the insertion process, the analyzer generates a pulse signal that changes from high level to low level and then from low level to high level and transmits it to the singlechip, signal 3 as shown in Fig. 26. The analyzer detects the falling signal of signal 3, and then determines that the detection device is already inserted and the analyzer is waked up. Immediately after that, the analyzer detects the rising signal of signal 3, and then determines that the detection device is already inserted in place. At the same time, the analyzer determines the matched type of the detection device based on the fact that signal 3 appears first as a falling signal and then as a rising signal. For example, we can set the detection device shown in Fig. 24 for drug detection.

The detection device shown in Fig. 25 includes two identification elements, wherein the first identification element includes a recessed portion 152 in the upper right corner, and the second identification element has a non-recessed portion 151 and a recessed portion 154 in the upper left corner in sequence, and the non-recessed portion 151 contacts the detection switch earlier than the recessed portion 154. In the process of inserting the detection device described in Fig. 25 into the analyzer, the first identification element, i.e., the upper right corner only includes a recessed portion, so that the detection switch will not be pressed, and the analyzer detects a signal that what is generated by the detection switch in the first identification area is signal 1. At the same time, the analyzer detects a signal that the detection switch, to which the second identification element (i.e., the non-recessed portion 151 and the other recessed portion 154 in the upper right corner) corresponds, generates signal 3. When the singlechip on the analyzer detects the two groups of signals with differences, it thus determines the matched type of the detection device. For example, we can set the detection device shown in Fig. 25 for analysis of estradiol.

## Claims

1. A detection device with identification information, comprising an upper plate and a bottom plate, which are assembled together to form the housing of the detection device, wherein an identification element for identifying the type of the detection device is disposed on the housing, and the identification element comprises a recessed portion, or a non-recessed portion, or a combination of the recessed portion and the non-recessed portion.

2. The detection device according to claim 1, comprising at least two identification elements, which are disposed at different positions on the detection device, respectively.

3. The detection device according to claim 1 or 2, wherein the identification elements are disposed at the front end or on the broadside of the housing of the detection device; or the identification elements are disposed at the front end and on the broadside of the housing simultaneously.

4. The detection device according to claim 3, wherein one of the two identification elements is set as a recessed portion, and the other one is set as a non-recessed portion; or the two identification elements are both set as recessed portions; or the two identification elements are both set as non-recessed portions.

5. The detection device according to claim 4, wherein one identification element is located in the upper right corner of the front end of the upper plate and is set as a recessed portion, and the other identification element is located in the upper left corner of the front end of the upper plate and is set as a non-recessed portion; or, one identification element is located in the upper right corner of the front end of the upper plate and is set as a non-recessed portion, and the other identification element is located in the upper left corner of the front end of the upper plate and is set as a recessed portion.

6. The detection device according to claim 1 or 2, wherein the identification element comprises at least one non-recessed portion and one recessed portion, wherein the non-recessed portion contacts a detection switch of the analyzer earlier than the recessed portion.

7. The detection device according to one of claims 1 to 6, wherein said identification element is used to trigger the detection switch in the analyzer to identify the type of the detection device.

8. The detection device according to claim 7, wherein the type of the detection device is identified in a way that the recessed portion does not trigger the detection switch and/or the non-recessed portion triggers the detection switch.

9. The detection device according to claim 7, wherein one identification element on the detection device corresponds to one detection switch on the analyzer.

10. The detection device according to claim 1, wherein the detection device further comprises test strip assembled between the upper plate and the bottom plate.

11. An analyzer for reading the identification information of a detection device, comprising an upper cover, a bottom cover, a detection device insertion port, and a circuit board assembled between the upper cover and the bottom cover, wherein the detection switch used to distinguish the type of the detection device is mounted to the circuit board; and the position of the detection switch in said analyzer corresponds to the position of the identification element of the detection device inserted into the analyzer.

12. The analyzer according to claim 11, wherein the detection switch in the analyzer is triggered by the non-recessed portion of the identification element of the detection device to enable the analyzer to power on and identify the type of the detection device.

13. The analyzer according to claim 12, wherein when the detection device is inserted into the analyzer, the non-recessed portion of the identification element of the detection device will touch the button for the detection switch and press the button to trigger the detection switch; and the recessed portion of the identification element of the detection device will not touch the button for the detection switch.

14. The analyzer according to claim 11, wherein the circuit board comprises two detection switches, which are in one-to-one correspondence with the two identification elements of the detection device, respectively.

15. The analyzer according to claim 11, wherein the side of the button for the detection switch is of a slope surface structure, the slop surface structure faces the detection device.

16. A method for identifying the type of a detection device, comprising a detection device and an analyzer, wherein the detection device comprises a housing formed by assembly of the upper plate and the bottom plate, the housing provided with an identification element, and the analyzer comprises an upper cover, a bottom cover, a detection device insertion port, a circuit board assembled between the upper and bottom covers, and a detection switch mounted to the circuit board to distinguish the type of the detection device, wherein through whether the detection switch of the analyzer is triggered by the identification element or not, the analyzer generates a signal corresponding to whether triggering is carried out or not to identify the type of the detection device.

17. The method for identifying the type of a detection device according to claim 16, wherein the identification element is provided with a non-recessed portion and a recessed portion, and through the fact that the recessed portion does not trigger the detection switch and/or the non-recessed portion triggers the detection switch, the analyzer generates a signal corresponding to whether triggering is carried out or not to identify the type of the detection device.

18. The method for identifying the type of a detection device according claim 17, wherein when the detection switch is not pressed by the recessed portion to switch on, the analyzer generates a signal 1; when the detection switch is pressed by the non-recessed portion to switch on, the analyzer generates a falling signal 2; and when the detection switch is first pressed by the non-recessed portion to switch on and then is switched off after losing the pressure of the non-recessed portion, the analyzer generates a signal 3 that rises first and then falls.

19. The method for identifying the type of a detection device according claim 16, wherein the analyzer is powered on or waked up from the dormant state through triggering the detection switch of the analyzer by the identification element.

20. The method for identifying the type of a detection device according claim 19, wherein when the detection switch is pressed by the non-recessed portion to switch on, the analyzer is powered on or waked up from the dormant state at the same time.

21. The method for identifying the type of a detection device according to any of claims 16 to 20, wherein the detection switch comprises a first detection switch and a second detection switch.

22. The method for identifying the type of a detection device according to claim 21, wherein the detection device comprises two identification elements, which correspond to the first detection switch and the second identification switch, respectively; one identification element is located in the upper right corner of the front end of the upper plate and is set as a recessed portion, and the other identification element is located in the upper left corner of the front end of the upper plate and is set as a non-recessed portion; or, one identification element is located in the upper right corner of the front end of the upper plate and is set as a non-recessed portion, and the other identification element is located in the upper left corner of the front end of the upper plate and is set as a recessed portion.

23. The method for identifying the type of a detection device according to claim 22, wherein the detection device is inserted into the analyzer, the recessed portion or non-recessed portion of the identification element travels to the detection switch and detects, the first detection switch and/or the second detection switch is pressed, the singlechip of the analyzer detects the type of the detection device; when both the first detection switch and the second detection switch are pressed, two falling signals 2 are sent, indicating that the detection device is a first type; or, when the first detection switch is pressed and the second detection switch is not pressed, a first falling signal 2 is sent, indicating that the detection device is a second type; or, when the first detection switch is not pressed and the second detection switch is pressed, a second falling signal 2 is emitted, indicating that the detection device is a third type; or, when neither the first detection switch nor the second detection switch is pressed, the signal 1 is emitted, indicating that the detection device is a fourth type.

24. The method for identifying the type of a detection device according to claim 23, wherein if the singlechip of the analyzer is in a dormant state before the detection device is inserted into the analyzer, the signal indicating the first or second detection switch is triggered will wake up the singlechip first, and then the singlechip will analyze the trigger signal from the detection switch, thus distinguishing the detection type of the detection device; if the singlechip is already in a wake-up state before the detection device is inserted, the singlechip will directly analyze the trigger signal from the detection switch, thus distinguishing the detection type of the detection device.

25. The method for identifying the type of a detection device according any of claims 21 to 24, comprising another identification element, which comprises a non-recessed portion and a recessed portion, wherein the non-recessed portion contacts the detection switch of the analyzer earlier than the recessed portion; the analyzer is provided with a detection switch corresponding to the identification element; and when the detection device advances in the analyzer, the detection switch is pressed first and then switched off, a signal 3 that first falls and then rises is sent, indicating that the detection device is of another type.
